# EUROPÄISCHE PATENTANMELDUNG

(11) **EP 0 780 476 A2**
(43) Veröffentlichungstag der Anmeldung: **25.06.1997**
(21) Anmeldenummer: 96115228.7
(22) Anmeldetag: 23.09.1996
(51) Int. Cl.: C12P 13/04

(54) **Verfahren zur Herstellung von L-Aminosäuren durch Fermentation**

(30) Priorität: 19.12.1995 DE 19547361
(71) Anmelder: Degussa Aktiengesellschaft, 60387 Frankfurt am Main (DE)
(72) Erfinder: Werning, Holger, 47057 Duisburg (DE); Voss, Harald, Prof. Dr. Dr., 8101 Gratkorn (AT); Pfefferle, Walter, Dr., 33790 Halle (DE); Leuchtenberger, Wolfgang, Prof. Dr., 33619 Bielefeld (DE)

(57) **Zusammenfassung**

Die Erfindung betrifft ein Verfahren zur Herstellung von L-Aminosäuren auf fermentativem Wege. Dabei kommen z. B. Mikroorganismen der Gattung Corynebacterium als Biokatalysator zur Anwendung, die bezüglich einer Aminosäure eine Auxotrophie besitzen. Das Verfahren ist dadurch gekennzeichnet, daß die Kohlenstoffquelle einerseits und die limitierende Aminosäure andererseits in zwei oder mehr verschiedenen Zufütterungsströmen dem Prozeß zugeführt werden. Dabei besitzen die Zufütterungsprofile beispielsweise eine konkave (Saccharose) und exponentielle (Aminosäure) Form oder eine konvexe (Saccharose) und ebenfalls konvexe (Aminosäure) Form, jeweils mit spezifischen unterschiedlichen Steigungsmaßen der Ströme zueinander.

## Beschreibung

Die Erfindung betrifft ein verbessertes Verfahren zur Herstellung von L-Aminosäuren durch Fermentation.

Von einer Reihe anderer Aminosäuren abgesehen, besteht z. B. für L-Lysin als essentieller Aminosäure in der Futter- und Nahrungsmittelindustre ein großer Bedarf. Die industrielle Produktion z. B. von L-Lysin durch fermentative Verfahren mit Mikroorganismen ist bekannt. Im allgemeinen werden dabei Mangelmutanten von Corynebacterium glutamicum verwendet. Diese Mutanten benötigen zum Wachstum einen der folgenden Nährstoffe:
Leucin oder Isoleucin, Homoserin, Threonin oder Threonin mit Methionin. Sie sind dann in der Lage, große Mengen von L-Lysin zu bilden.

Übliche *Fed*-*Batch*-Prozesse werden daher so gesteuert, daß neben anderen Nährstoffen und Spurenelementen sowohl die wachstumslimitierende Aminosäure als auch die für die L-Lysinbildung notwendige Kohlenstoffquelle kontinuierlich zugefüttert wird.

Es wurde jetzt gefunden, daß diese Prozeßführung, bei der die C-Quelle, wie z. B. Saccharose und die limitierende(n) Aminosäure(n) während der Wachstums- und Fermentationsphase in einem zueinander konstanten Konzentrationsverhältnis zugefüttert werden, zur Bildung unerwünschter Nebenprodukte und zum Schäumen der Fermentationsbrühe während des Prozesses führen.

Gleichzeitig wurde nach einem Weg gesucht, die Produktivität bzw. die Reaktorleistung zu steigern.

Gegenstand der Erfindung ist ein Verfahren zur Herstellung von L-Aminosäuren durch Fermentation von L-Aminosäuren ausscheidende Mikroorganismen, die bezüglich mindestens einer anderen Aminosäure eine Auxotrophie besitzen, das dadurch gekennzeichnet ist, daß man die für das Wachstum und die L-Aminosäurebildung nötige Kohlenstoffquelle einerseits und eine das Wachstum bzw. die Aminosäurebildung limitierende(n) Aminosäure(n) zusammen mit einer adäquaten Menge anderer Nährstoffe und Spurenelemente andererseits in zwei oder mehr getrennten zeitlich aber gleich einsetzenden Zulaufströmen mit zeitlichen Zufütterungsprofilen unterschiedlicher Form zudosiert, wobei die Dosierung bevorzugt nicht in proportionalen Mengen erfolgt.

In einer bevorzugten Ausführungsform weist der Zulaufstrom, der die limitierende(n) L-Aminosäure(n) enthält, in der graphischen Darstellung eine exponentielle Form, der Zulaufstrom aber, der die Kohlenstoffquelle enthält, ein bezüglich der Zeitachse konkaves Profil auf, insbesondere beginnend mit einem stelen Anstieg, der vor dem konkaven Profil liegt.

In einer weiteren Ausführungsform ergibt sich folgendes Bild, daß der Zulaufstrom, der die limitierende L-Aminosäure enthält, in der graphischen Darstellung einen Anstieg entsprechend einer konvexen Form und der Zulaufstrom, der die Kohlenstoffquelle enthält, ein bezüglich der Zeitachse konkaves Profil aufweisen, wobei beide Profile bis zu Ende der Fermentation eine unterschiedliche Steigung zeigen, in dem Sinne, daß die Steigung des Kohlenstoffquellenzulaufs größer ist, wie allgemein gilt.

Eine weitere Variante zeichnet sich dadurch aus, daß der Zulaufstrom, der die limitierende L-Aminosäure enthält, eine konvexe Form und der Zulaufstrom, der die Kohlenstoffquelle enthält, ebenfalls ein bezüglich der Zeitachse konvexes Profil aufweist, wobei beide Profile unterschiedliche Steigungsmaße zeigen.

Als günstig hat sich auch die Kombination einer linear fallenden Zufütterungsrate der Kohlenstoffquelle mit einem konvex verlaufenden Zulaufstrom der limitierenden Aminosäure erwiesen.

Bei den herzustellenden L-Aminosäuren handelt es sich um Verbindungen aus der Gruppe entsprechend Anspruch 6, insbesonder L-Lysin.

Zur Herstellung verwendete Mikroorganismen stammen aus den Gattungen , wie sie in Anspruch 7 aufgeführt werden.

Bevorzugt eingesetzt wird die beschriebene Vorgehensweise zur Fermentation von L-Lysin unter Verwendung diese Aminosäuren ausscheidender Mikroorganismen der Gattungen Corynebacterium, Brevibacterium, Arthrobacter, Microbacterium, Bacillus oder Nocordia.

In einer speziellen Ausführungsform der Erfindung zeigt sich, daß insbesondere bei auxotrophen Stämmen von Corynebacterium glutamicum im Rahmen einer halbkontinuierlichen Fermentation eine hohe L-Lysin-Produktivität erreicht wird, wenn man für eine spezifische Produktbildungsrate von 0.06 h⁻¹ bis 0.25 h⁻¹ einstellt.

Nach dem erfindungsgemäßen Verfahren sind zur fermentativen Herstellung von L-Lysin Mikroorganismen der Gattungen Corynebacterium, Brevibacterium, Arthrobacter, Microbacterium, Bacillus oder Nocardia verwendbar, die sich gegenüber einer oder mehreren der L-Aminosäuren Leucin, Homoserin, Threonin, Methionin, Isoleucin auxotroph verhalten und gegen Rückkopplungshemmung und/oder Repression durch mindestens eine der Verbindungen Lysin und Lysin-Analoge, Threonin und Threonin-Analoge und Isoleucin und Isoleucin-Analoge resistent sind.

Ein Vorteil der erfindungsgemäßen Verfahrensstrategie gegenüber bekannten Verfahren mit nur einem Zulaufstrom ist die signifikante Steigerung der Produktivität der Mikroorganismen, z. B. besonders im Hinblick auf das L-Lysin.

Ein Vergleich der volumetrischen Produktivitäten (s. Beispiele) führt zu einer Verbesserung von >50 %. Auch die füllgradunabhängig vorgenommene Berechnung der Reaktorleistung zeigt deren Erhöhung um >10 %.

### Beispiele

### Beispiel 1

Dieser Prozeßablauf erläutert die Prozeßführung einer L-Lysin-Fermentation mit besonders hoher Reaktorleistung:

In zwei Propagationsstufen wird der Stamm Corynebacterium glutamicum DM282-2 zu 2 l Inokulum herangezüchtet. Dazu wird jeweils ein Anzuchtmedium verwendet, das 1.92 % Saccharose, 4.19 % Melasse, 11.55 % Sojamehlhydrolysat, 3.05 % Ammoniumsulfat, 0.58 % Harnstoff, 0.024 % Magnesiumsulfat-Heptahydrat, 0.05 % KH₂PO₄, 0.06 % Citronensäure-Monohydrat, 0.00096 % Eisen(II)-Sulfat-Heptahydrat, 0.00096 % Mangan(II)-sulfat-Monohydrat, 0.00577 % L-Leucin, 0.00385 % L-Threonin, 0.0077 % L-Methionin, 0.000038 % D-Biotin, 0.19 % Thiamin·Hcl und 0.96 % Nalco enthält. Es wird ein pH-Wert von 6.8 mit NaOH eingestellt. Mit dem so gewonnenen Inokulum wird eine Propagations-Fermentation beimpft, die 300 l des Fermentationsmediums M1 enthält. Dieses Medium besteht aus 0.51 % Melasse, 7.69 % Saccharose, 5.69 % Maiskleberhydrolysat, 1.15 % Ammoniumsulfat, 0.0029 % Mangan(II)-sulfat-Monohydrat, 0.072 % Magnesiumsulfat-Heptahydrat, 0.0029 % Eisen(II)-sulfat-Heptahydrat, 0.001923 % Kalziumchorid-Dihydrat, 0.0015 % Zinksulfat-Heptahydrat, 0.000014 % Kupfersulfat-Heptahydrat, 0.0577 % Citronensäure-Monohydrat, 0.01 % H₃PO₄, 0.000029 % D-Biotin, 0.000019 % Thiamin-HCl, 0.096 % Nalco, 0.000048 % Ferrioxamin E, FeCl₃. Dieses Gemisch wird ca. 20 Stunden bei 30 °C, pH 7.0 und einem geregelten pO₂ von mindestens 15 % kultiviert. Dabei wird eine Biomassenkonzentration von etwa 10 g/l erreicht.

Mit diesen 300 l Fermentationsbrühe aus der Propagations-Fermentation wird nun der Produktions-Fermenter beimpft, der 4000 l Medium M1 enthält. Bis zum Erreichen einer optischen Dichte von OD 30 wird diese Fermentation bei 33 °C, pH 7.1 und pO₂ >15 % geführt.

Dann erfolgt mit dem Start der Zufütterung eine Erhöhung der Temperatur auf 35 °C und des pH-Wertes auf pH 7.5.

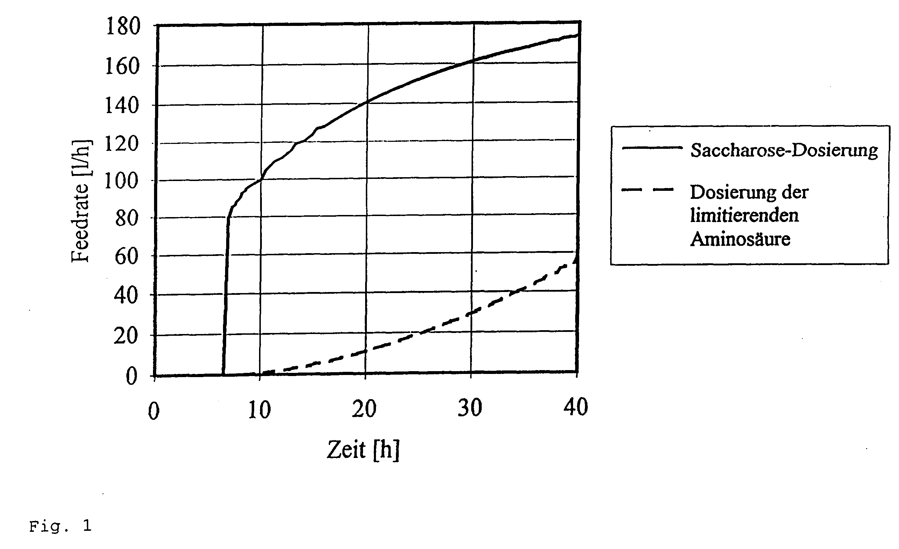

Die Zufütterungsmedien M2.1 und M2.2 werden wie in Fig. 1 dargestellt. zudosiert.
Dabei besteht das Zufütterungsmedium M2.1 (Saccharose-Dosierung) aus einer Saccharoselösung von 600 g/l und das Zufütterungsmedium M2.2 (Aminosäure-Dosierung) aus einer Lösung, die 15 g/l limitierende Aminosäure und die oben erwähnten Mineral- und Zusatzstoffe in adäquater Menge enthält.

### Beispiel 2

Dieser Prozeßablauf erläutert die Prozeßführung einer L-Lysin-Fermentation mit besonders hoher Produktivität der Mikroorganismen bezüglich L-Lysin:

Die Medienzusammensetzung und die Animpfprozedur erfolgt wie bei Beispiel 1.
Bis zu Erreichen einer optischen Dichte von OD 30 wird diese Fermentation bei 33 °C, pH 7.1 und pO₂ >15 % geführt.

Dann erfolgt mit dem Start der Zufütterung eine Erhöhung der Temperatur auf 35 °C und des pH-Wertes auf pH 7.5.

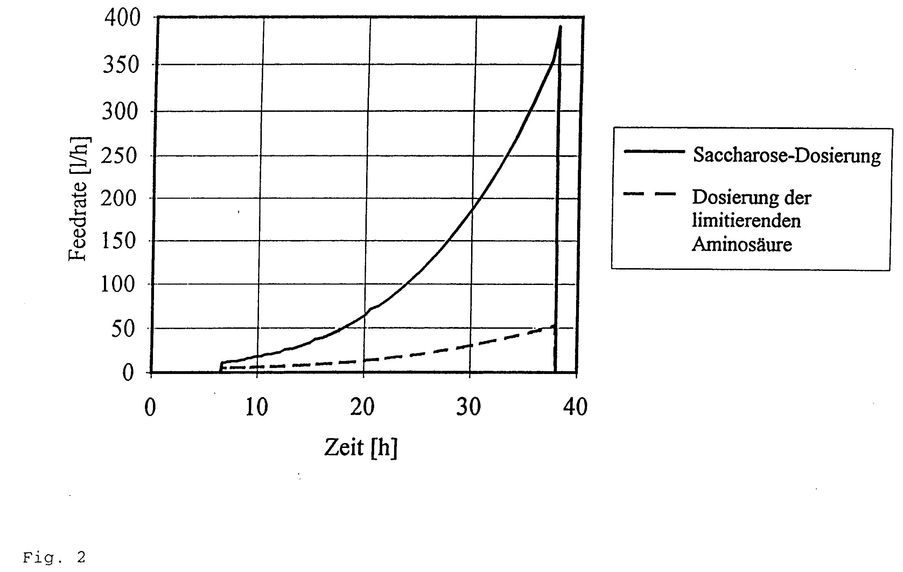

Die Zufütterungsmedien M2.1 und M2.2 werden wie in Fig. 2 dargestellt, zudosiert.

Dabei besteht das Zufütterungsmedium M2.1 (Saccharose-Dosierung) aus einer Saccharoselösung von 600 g/l und das Zufütterungsmedium M2.2 (Aminosäure-Dosierung) aus einer Lösung, die 15 g/l limitierende Aminosäure und die oben erwähnten Mineral- und Zusatzstoffe in adäquater Menge enthält.

### Beispiel 3

Dieses Beispiel beinhaltet den Vergleich mit einem Standardversuch, bei dem Kohlenstoffquelle und limitierende Aminosäure (L-Leucin) in einem Strom angefüttert werden.

Die Medien entsprechen ebenso wie die sonstigen Verfahrensmaßnahmen im Grundsatz denen aus den Beispielen 1 und 2.

| Parameter | 941147 Standard mit 1 Zulauf | 941177 Erfindungsgemäß |
|---|---|---|
| Inokulum (l) | 0,38 | 0,38 |
| Startvolumen (l) | 5,78 | 2,78 |
| Zulauf Medium (l) | 2,64 (Zucker+Supplemente) | 2,13 (Zucker)(M 2.1) |
| verbrauchte Saccharose in g | 2058 | 2317 |
| Zulauf Medium 2 (l) | - | 0,86 (Supplemente)(M2.2) |
| Endkonz. Lys (g/l) | 46,46 | 69,29 |
| Ferm.-zeit (h) | 30,5 | 30 |
| Fermentationsendvolu men (l) | 8,60 | 6,320 |
| Reaktorleistung (g Lysin/h) | 13,1 | 14,6 |
| Produktivität (g Lysin/l*l) | 1,51 | 2,303 |

Es findet sich eine volumetrische Produktivitätssteigerung von 52,4 % (aus der Berechnung 2,303 : 1,51). Die füllgradunabhängige Berechnung führt zu einer Leistungssteigerung des Reaktors um 11,6 % (aus der Berechnung 14,6 : 13,1)

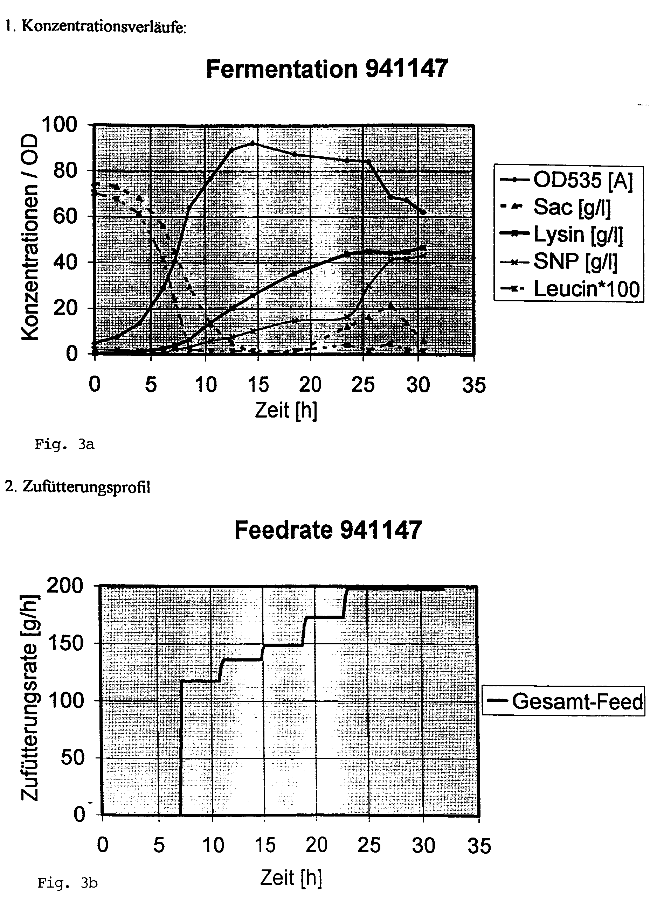

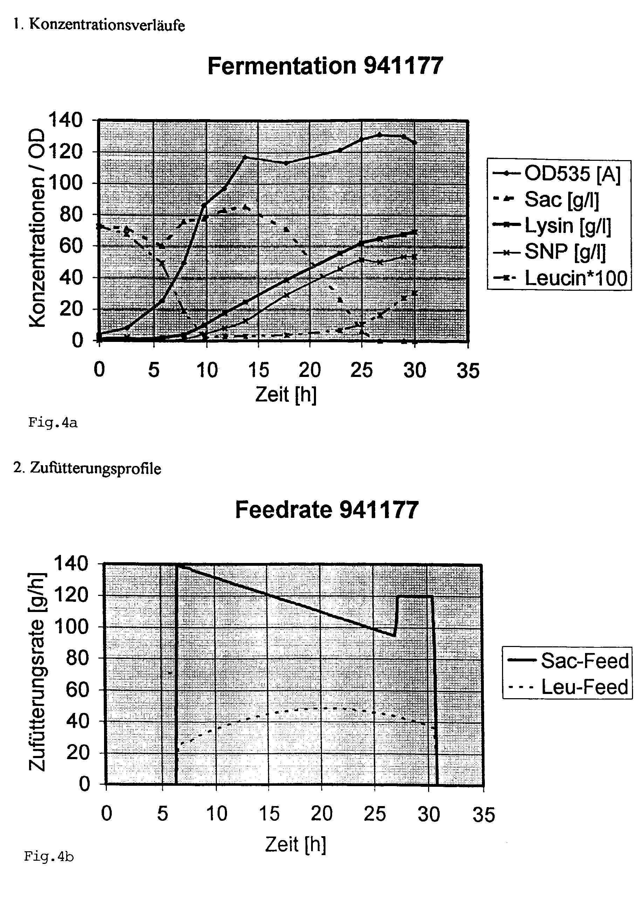

Die Abbildungen geben die Verläufe der Fermentation und der Zulaufströme wieder (Fig. 3a,3b; Fig.4a, 4b).

## Patentansprüche

1. Verfahren zur Herstellung von L-Aminosäuren durch Fermentation von L-Aminosäuren ausscheidenden Mikroorganismen, die bezüglich mindestens einer anderen Aminosäure eine Auxotrophie besitzen,
dadurch gekennzeichnet, daß man die für das Wachstum und die L-Aminosäurebildung nötige Kohlenstoffquelle einerseits und eine der das Wachstum bzw. die Aminosäurebildung limitierende(n) Aminosäure(n) zusammen mit einer adäquaten Menge anderer Nährstoffe und Spurenelemente andererseits in zwei oder mehr getrennten Zulaufströmen mit zeitlichen Zufütterungsprofilen unterschiedlicher Form zudosiert.

2. Verfahren gemäß Anspruch 1,
dadurch gekennzeichnet, daß der Zulaufstrom der die limitierende(n) L-Aminosäure(n) enthält, in der graphischen Darstellung eine exponentielle Form, der Zulaufstrom, der die Kohlenstoffquelle enthält, ein bezüglich der Zeitachse konkaves Profil aufweist.

3. Verfahren gemäß Anspruch 1,
dadurch gekennzeichnet, daß der Zulaufstrom, der die limitierende(n) L-Aminosäure(n) enthält, in der graphischen Darstellung eine konvexe Form und der Zulaufstrom, der die Kohlenstoffquelle enthält, ein bezüglich der Zeitachse konkaves Profil aufweist, wobei beide Profile unterschiedliche Steigungsmaße zeigen.

4. Verfahren gemäß Anspruch 1,
dadurch gekennzeichnet, daß der Zulaufstrom, der die limitierende(n) L-Aminosäure(n) enthält, in der graphischen Darstellung eine konvexe Form und der Zulaufstrom, der die Kohlenstoffquelle enthält, ebenfalls ein bezüglich der Zeitachse konvexes Profil aufweist, wobei beide Profile unterschiedliche Steigungsmaße zeigen.

5. Verfahren gemäß Anspruch 1,
dadurch gekennzeichnet, daß der Zulaufstrom, der die Kohlenstoffquelle enthält, einer linear fallenden Linie entspricht, während die Zufütterung an limitierender Aminosäure einem konvexen Profil entspricht, beides bezogen auf die Zeitachse.

6. Verfahren gemäß den Ansprüchen 1 bis 5, dadurch gekennzeichnet, daß man eine L-Aminosäure aus der Gruppe
L-Lysin,
L-Methionin,
L-Aspartat
L-Asparagin
L-Isoleucin
L-Threonin
herstellt.

7. Verfahren gemäß Anspruch 6,
dadurch gekennzeichnet, daß man Mikroorganismen der Gattungen:
Corynebacterium sp., Brevibacterium sp., Arthrobacter sp.,Corynebacterium glutamicum, Bacillus sp., Brevibacterium flavum,Brevibacterium lactofermentum, Microbacterium oder Nocordia
einsetzt.

8. Verfahren gemäß den Ansprüchen 6 und 7
dadurch gekennzeichnet, daß man L-Lysin unter Verwendung von Aminosäuren-auxotrophen Stämmen der Gattungen Corynebacterium, Brevibacterium, Arthrobacter, Microbacterium, Bacillus oder Nocordia herstellt.

9. Verfahren gemäß Anspruch 8,
dadurch gekennzeichnet, daß die Stämme auxotroph in Bezug auf eine oder mehrere der L-Aminosäuren Leucin, Homoserin, Threonin, Methionin, Isoleucin sind und/oder Resistenz gegen mindestens eine der Verbindungen L-Lysin oder Lysin-Analoga, Isoleucin oder Isoleucin-Analoga oder Threonin oder Threonin-Analoga aufweisen.

10. Verfahren gemaß den Ansprüchen 8 und 9,
dadurch gekennzeichnet, daß eine spezifische Produktbildungsrate von 0.06 h⁻¹ bis 0.25 h⁻¹ eingestellt wird.
